(19) Europäisches Patentamt
European Patent Office
Office européen des brevets

(11) Veröffentlichungsnummer: **0 187 345**
**B1**

(12) # EUROPÄISCHE PATENTSCHRIFT

(45) Veröffentlichungstag der Patentschrift: **21.09.88**

(21) Anmeldenummer: **85116323.8**

(22) Anmeldetag: **20.12.85**

(51) Int. Cl.⁴: **C 07 D 261/04**, C 07 D 307/34, C 07 F 9/65 // C07D307/42, C07D307/68, C07C49/17, C07C49/245

(54) **Substituierte Furane und deren Vorprodukte.**

(30) Priorität: **29.12.84 DE 3447793**
**20.04.85 DE 3514384**

(43) Veröffentlichungstag der Anmeldung: **16.07.86 Patentblatt 86/29**

(45) Bekanntmachung des Hinweises auf die Patenterteilung: **21.09.88 Patentblatt 88/38**

(84) Benannte Vertragsstaaten: **AT BE CH DE FR GB IT LI NL SE**

(56) Entgegenhaltungen:
**TETRAHEDRON LETTERS, Band 24, Nr. 20, 1983, Seiten 2079-2082, Pergamon Press Ltd., Oxford, GB; D.P. CURRAN et al.: "Reduction of delta2-isoxazolines-2. A facile synthesis of 3(2H)-furanones"**
**TETRAHEDRON LETTERS, Band 24, Nr. 49, 1983, Seiten 5501-5504, Pergamon Press Ltd., Oxford, GB; V. JÄGER et al.: "Asymmetric induction in nitrile oxide cycloadditions to 3-butene-1,2-diol and derivatives"**
**ACTA CHEMICA SCANDINAVICA, Band B 36, 1982, Seiten 1-14, Copenhagen, DK; S.H. ANDERSEN et al.: "Silyl nitronates in organic synthesis. Routes to heterocycles and cyclopentanoids. Synthesis of allethrolone and calythrone. Acylation and cyanohydroxylation of double bonds. An exploratory study"**

(73) Patentinhaber: **BASF Aktiengesellschaft, Carl-Bosch- Strasse 38, D-6700 Ludwigshafen (DE)**

(72) Erfinder: **Theobald, Hans, Dr., Queichstrasse 6, D-6703 Limburgerhof (DE)**
Erfinder: **Becker, Rainer, Dr., Im Haseneck 22, D-6702 Bad Duerkheim (DE)**
Erfinder: **Himmele, Walter, Dr., Eichenweg 14, D-6909 Walldorf (DE)**

**EP 0 187 345 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Bis zu vierfach substituierte Furane der allgemeinen Formel (I)

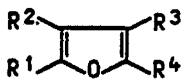

(I)

in der

$R^1$ bis $R^4$ Wasserstoff oder unsubstituiertes oder durch Halogen, Haloalkyl, Alkoxy, Haloalkoxy, Nitro, Cyano, Dialkylphosphonyl oder Alkoxycarbonyl substituiertes Alkyl, Aryl, Aralkyl, Alkoxycarbonyl oder Dialkoxyphosphonyl,

$R^3$ auch Hydroxyalkyl oder durch eine abspaltbare Schutzgruppe verkapptes Hydroxyalkyl bedeuten, werden als Vorprodukte, z. B. für die Herstellung von Pflanzenschutzmitteln benötigt: Es ist bekannt, daß Furane aus 1,4-Diketonen durch Cyclisierung hergestellt werden können (Comprehensive Heterocyclic Chemistry Vol. 4 657 ff, (1984). Bei der Synthese von insbesondere asymmetrisch substituierten Furanen (z. B. 2,4-Disubstitution) ist die Herstellung der erforderlichen Ausgangsprodukte schwierig. Aus diesem Grunde werden beispielsweise für die Herstellung von 2-Benzyl-4-hydroxymethylfuran, einem gesuchten Zwischenprodukt für Insektizidsynthesen, verschiedene Verfahren empfohlen (JCS (c) 1971, 2551; Wegler Vol. 7 (1981) S. 76; DOS 2 122 823). Alle diese Verfahren weisen jedoch schwerwiegende Nachteile auf (vielstufige Synthesen mit mäßiger Gesamtausbeute, Sicherheitsprobleme durch hohe Temperaturen, Isomerenbildung).

Es ist eine Aufgabe der Erfindung, ein Verfahren zur Herstellung substituierter Furane anzugeben, das mit leicht zugänglichen Ausgangsstoffen auskommt und leicht durchzuführen ist.

Es wurde gefunden, daß man Furane der vorgenannten Art vorteilhaft dadurch erhält, daß man ein entsprechendes Isoxazolin der Formel II

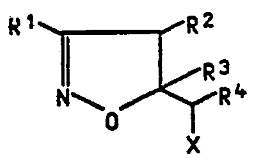

(II),

in der X Hydroxy, Halogen oder durch eine abspaltbare Schutzgruppe verkappter Hydroxy bedeutet, hydriert.

Die Hydrierung substituierter Isoxazoline ist prinzipiell bekannt (J. Am. Chem. Soc. 104, 4024 (1982) Tetrahedron Lett. 23, 3123 (1982) und 24, 1337 (1983)) und wurde auch zur Heterocyclensynthese genutzt. So lassen sich beispielsweise aus geeignet substituierten Isoxazolinen durch hydrierende Spaltung Pyrone (US-Pat 4 136 114), Pyridine (J. Org. Chem. 36, 2784 (1971)) Pyrimidine bzw. Pyrimidone (J. Pharm. Soc. (Jap) 83, 471 (1963) oder Ann. Chimica 60, 393 (1970)), Pyrrole (Ann. Chimica 56, 858 (1966)), Pyrazole (Chem. Ber. 106, 332 (1973) oder TH 29, 4291 (1973)), Imidazole (Ann. Chimica 60, 343 (1970)) oder auch Furanone (Tetrahedron Lett. (1983) 2079 oder (1966) 233 oder (1967) 327 bzw. Gazetta (1966) 1073) erhalten.

Die Bildung der erfindungsgemäßen Furane wird bei keiner dieser Hydrierungen beobachtet, obwohl geeignet substituierte Ausgangsprodukte (d. h. 5-hydroxymethylsubstituierte Isoxazoline - z. B. J. Ann. Chem. Soc. 105, 5826 - 5833 (1983); Verbindung 18 a) - durchaus in diese Reaktion eingesetzt wurden. Lediglich eine bei Acta chem. Scand B 36, 1 - 14 (1982) beschriebene sauer katalysierte Umlagerung von Isoxazolin unter nichthydrierenden Bedingungen ergab für eine spezielle Substitution über eine Diketon-Zwischenstufe ein Furan-Derivat. Leicht abweichende Substitutionsmuster gaben keine Furanderivate mehr, vielmehr Furanone bzw. Cyclopentenone.

Die Hydrierung liefert in saurer Umsetzung unmittelbar die gewünschten Furane. Unter nicht-sauren Bedingungen entsteht zunächst ein entsprechendes offenkettiges Hydroxyketon, dem die allgemeine Formel III

$$R^1CO\text{-}CHR^2\text{-}C(OH)R^3\text{-}CH(X)R^4$$

zukommt, das als Zwischenprodukt isoliert werden kann und beim Ansäuren in ein Furan der allgemeinen Formel I übergeht.

Isoxazoline der allgemeinen Formel II, außer solchen, in denen $R^1$ oder $R^3$ Wasserstoff bedeutet, sind neue Stoffe. Es versteht sich, daß in den Fällen, wo sowohl $R^3$ ein verethertes oder verestertes Hydroxymethyl als auch X verkapptes Hydroxy bedeutet, eine Veretherung bzw. Veresterung mit einem jeweils bifunktionellen Partner unter Ringbildung stattgefunden haben kann Abhängig von der speziellen Verfahrensweise kann dies direkt geschehen oder es kann als Zwischenstufe das Hydroxyketon III

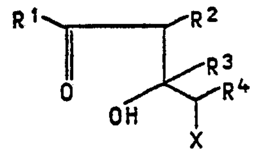

(III),

isoliert und in einem anschließenden Schritt das gewünschte Furanderivat I erhalten werden:

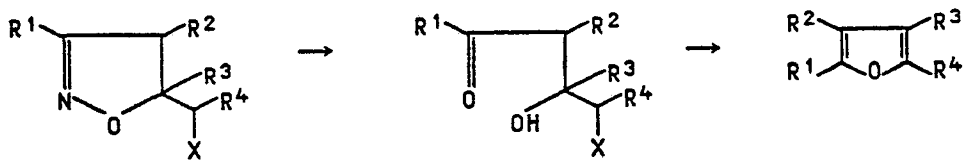

(II) (III) (I)

Für den Fall des oben erwähnten 2-Benzyl-4-hydroxymethylfurans läßt sich die Umsetzung wie folgt wiedergeben:

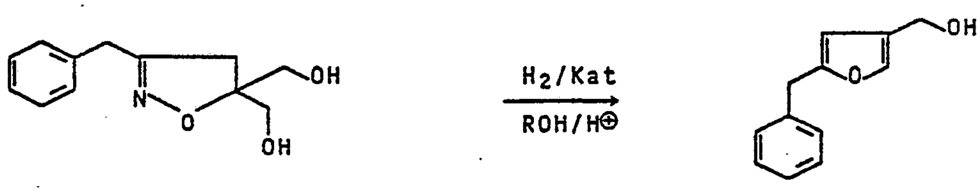


Überraschend ist der glatte Verlauf dieser Reaktion u. a. auch deshalb, weil bislang durch Hydrierung speziell substituierter Isoxazole lediglich die Synthese von Dihydrofuran-3-onen beschrieben ist (THL (1983) 2079; THL (1966) 233; THL (1967) 327; Gazetta (1966) 1073):

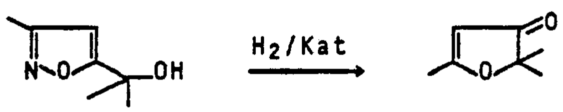


Weiterhin lieferte eine spezielle Isoxazolinsubstitution bei der Reduktion mit $TiCl_3$ 2,5-disubstituierte Furane (Acta Chem. Scand B 36 (1982):

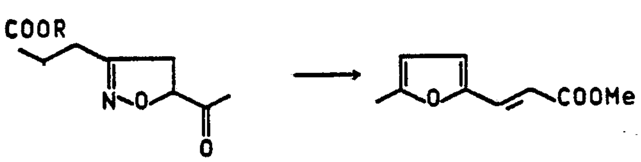

Zur Herstellung der neuen Vorprodukte II ist insbesondere das folgende zu sagen:
Isoxazoline II

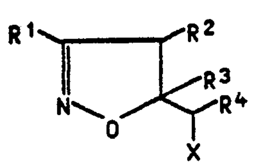

(II)

können z. B. nach der Methode in Heterocycles Bd. 12, S. 1243 ff. (1979) durch Umsetzen von Nitriloxiden mit bestimmten Olefinen erhalten werden nach

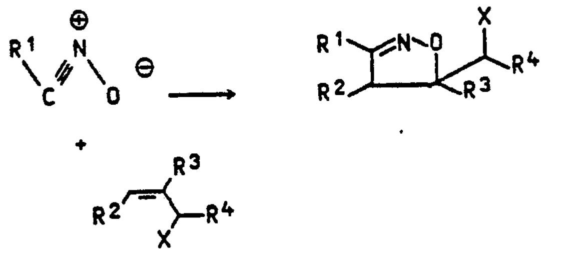

(II)

In der vorstehenden Umsetzung bedeutet X Hydroxy oder durch eine abspaltbare Schutzgruppe, z. B. t-Butyl, Trimethylsilyl, Acetyl, Benzyl, verkapptes Hydroxy. Es lassen sich im übrigen manche der erfindungsgemäßen Isoxazoline aus anderen, nach der vorstehenden Methode zugänglichen Isoxazolinen erhalten: z. B. kann in 3-Stellung substituiertes 5,5-Bis-hydroxy-methylisoxazolin an den Hydroxygruppen mit den vorgenannten Schutzgruppen versehen oder diese abgespalten werden oder z. B. Hydroxy und Halogen oder Halogen und eine Schutzgruppe gegeneinander ausgetauscht werden.

Die als Ausgangsstoffe benötigten Nitriloxide können ihrerseits z. B. aus entsprechenden Oximen erhalten werden oder aus aliphatischen Nitroverbindungen.

Es wird hierzu auf Heterocycles, a. a. O. und die dort aufgeführten Literaturhinweise, ferner auf Houben-Weyl, Methoden, 14. Aufl. Band 10/3, S. 83 und auf J. Org. Chem. 28, 1150 (1963) verwiesen. Ober spezielle Aspekte der asymmetrischen Induktion bei dieser Isoxazolinsynthese berichten Jäger und Schohe (Tetrahedron Lett. 24, 5501 - 04 (1983).

Die Isolierung der Nitriloxide der Formel II ist nicht unbedingt erforderlich. Sie werden vorteilhaft gleich in Anwesenheit der Alkenylverbindungen erzeugt, mit denen sie sich ummittelbar zu den Isoxazolinen der Formel II umsetzen.

Die zur Darstellung der Isoxazoline verwendeten Nitriloxide und die Alkenylverbindungen können in äquimolaren Verhältnissen oder jeweils einer der Reaktionspartner im Überschuß eingesetzt werden.

Als Lösungsmittel für die Umsetzung eignet sich z. B. die jeweilige Alkenylverbindung selbst, ferner aromatische Verbindungen (z. B. Benzol, Toluol, Xylol), halogenierte aromatische Verbindungen, Ketone (z. B. Aceton, Methylethylketon, Diisopropylketon), Ether (z. B. Dioxan, Diethylether, THF), chlorierte aliphatische Kohlenwasserstoffe (z. B. Dichlorethan, Chloroform, Methylenchlorid).

Es ist darauf hinzuweisen, daß die Anlagerung der Olefine an diese Nitriloxide je nach der Art der Substituenten verläuft und daher u. U. Isomere bzw. Gemische von Isomeren erwartet werden können. Als allgemeine Regel ist anzunehmen, daß die an das Sauerstoffatom des Nitriloxids angelagerte Seite der olefinischen Bindung diejenige ist, die den - räumlich - größeren Substituenten trägt.

Alle vorstehend beschriebenen Umsetzungen verlaufen gewöhnlich unterhalb von etwa 150°C mit ausreichender Geschwindigkeit.

**Herstellbeispiel 1 für ein Isoxazolin**

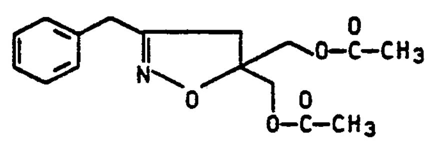

159 g Phenylacetaldoxim und 202,5 g Isobutylendiacetat werden in 500 ml Diethylether gelöst und unter starkem Rühren bei 10 bis 15°C mit 1 050 ml einer 10-%-igen Natriumhypochloritlösung versetzt. Nach mehrstündigem Rühren wird die organische Phase mit Wasser ausgeschüttelt über Natriumsulfat getrocknet und vom Lösungsmittel befreit.

Ausbeute 333 g ≙ 92,8 % der berechneten Menge. Kristalle aus Ethanol-Petrolether schmelzen bei 78 bis 79°C.

**Herstellbeispiel 2 für ein Isoxazolin**

Zu 200 g Allylacetat und 242 g 2-Methylbutyraldoxim in 600 g Methylenchlorid werden 1960 g 10-%-ige NaOH-Lösung nach und nach zugegeben und 3 Stunden bei Raumtemperatur gerührt. Die organische Phase

wird abgetrennt, mit Wasser gewaschen, über $Na_2SO_4$ getrocknet und nach Filtration vom Lösungsmittel befreit. Das zurückbleibende Öl (339 g) ist 3-sec.Butyl-5-acetoxymethyl-isoxazol.

300-MHz-NMR-Spektrum in $CDCl_3$ (ppm): 0,95 (3H); 1 ,08 (3H); 1,2 - 1,3 (2H); 2,04 (3H); 2,3 (1H); 2,35 (1H); 3,0 (1H); 4,0 - 4,1 (2H); 4,4 (1H).

**Herstellbeispiel 1 für ein Furan**

88,4 g 3-Benzyl-5,5-bis(hydroxymethyl)isoxazolin werden in 550 ml Tetrahydrofuran, 40 ml Wasser und 80 ml Eisessig aufgenommen und mit 1,6 g Platinoxid versetzt. Beim Einleiten von Wasserstoff setzt eine leicht exotherme Reaktion ein; nach beendeter Wasserstoffaufnahme wird abfiltriert, eingeengt und der Rückstand mit Aceton verrührt. Das ungelöste Ammoniumacetat wird abfiltriert, das Filtrat wieder eingeengt kund dann in 300 ml Hethylenchlorid mit 300 ml 10-%-iger Salzsäure eine Stunde bei 20°C verrührt. Dann wird die Lösung in Methylenchlorid abgetrennt, mit Wasser gewaschen und eingeengt. Es verbleiben 59,2 g 2-Benzyl-4-hydroxymethylfuran NMR-spektroskopisch praktisch rein (79 % bezogen auf Isoxazolin). Destillation (0,3 mm/125 - 128° liefert 52,0 g einer Substanz mit einem Schmelzpunkt von 37 bis 38°C).

**Herstellbeispiel 2 für ein Furan**

100 g 3-sec.Butyl-5-acetoxymethylisoxazol in 400 g Methanol und 27 g Wasser werden mit 25 g Raney-Nickel bei 20 - 30° hydriert. Danach wird filtriert, vom Lösungsmittel befreit und destilliert. Die bei 70 - 80°C überdestillierende Flüssigkeit ist 2-sec. Butyl-furan. 200 MHz-NMR-Spektrum in $CDCl_3$ (ppm) : 0,9 (3H); 1,25 (2H); 1,5 - 1,8 (2H); 2,78 (1H); 6,0 (1H); 6,3 (1H); 7,35 (1H).

Nach den vorstehenden Vorschriften können aus entsprechenden Ausgangsstoffen weitere Isoxazoline hergestellt werden. So wurden die in der nachstehenden Tabelle aufgeführten Stoffe hergestellt, die eine Vorstellung von den vielseitigen Möglichkeiten des erfindungsgemäßen Verfahrens und der damit erhältlichen Wirkstoffe bzw. deren Vorprodukte vermitteln.

| $R^1$ | $R^2$ | $R^3$ | $R^4$ | X | |
|---|---|---|---|---|---|
| $CH_3$ | H | $CH_3-\overset{O}{\overset{\Vert}{C}}-OCH_2$ | H | $CH_3-\overset{O}{\overset{\Vert}{C}}-O$ | $Kp_{0,25}$ =133-136°; $M_{D25}$ =1,4572 |
| $C_2H_5-OOC$ | H | $CH_3-\overset{O}{\overset{\Vert}{C}}-OCH_2$ | H | $CH_3-\overset{O}{\overset{\Vert}{C}}-O$ | 80 MHz-NMR in $CDCl_3$(ppm): 1,4(3H); 2,15(6H); 3,2(2H); 4,2-4,6(6H); |
| 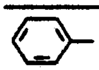 | H | $CH_3-\overset{O}{\overset{\Vert}{C}}-OCH_2$ | H | $CH_3-\overset{O}{\overset{\Vert}{C}}-O$ | Fp = 79-80°C |
| 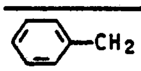 | H | $CH_3-\overset{O}{\overset{\Vert}{C}}-OCH_2$ | H | $CH_3-\overset{O}{\overset{\Vert}{C}}-O$ | Fp = 78-79°C |
| 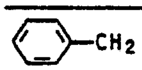 | H | $CH_2-OH$ | H | OH | Fp = 68-74°C |
| $CH_3-OCH_2$ | H | $CH_3$ | H | $CH_3-\overset{O}{\overset{\Vert}{C}}-O$ | $Kp_{0,2}$ =107-110°C |
| 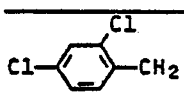 | H | $CH_3-\overset{O}{\overset{\Vert}{C}}-OCH_2$ | H | $CH_3-\overset{O}{\overset{\Vert}{C}}-O$ | 200 MHz-NMR in $CDCl_3$ (ppm) 2,06(6H); 2,8(2H); 3,81(2H) 4,16(4H); 7,25(2H); 7,43(1H). |
| 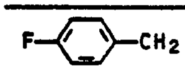 | H | $CH_2OH$ | H | OH | Fp = 93-97°C |
| 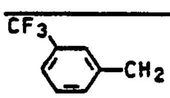 | H | $CH_3-\overset{O}{\overset{\Vert}{C}}-OCH_2$ | H | $CH_3-\overset{O}{\overset{\Vert}{C}}-O$ | 300-MHz-NMR in $CDCl_3$ (ppm) 2,01(6H); 2,8(2H); 3,75(2H); 4,04-4,23(6H); 7,4-7,68(4H). |
| 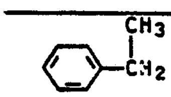 | H | $CH_3-\overset{O}{\overset{\Vert}{C}}-OCH_2$ | H | $CH_3-\overset{O}{\overset{\Vert}{C}}-O$ | 300-MHz-NMR in $CDCl_3$ (ppm) 1,53(3H); 2,0(3H); 2,03(3H); 2,71(2H); 3,82(1H); 4,12(2H); 4,15(2H). |

**Patentansprüche**

1. Isoxazolin der allgemeinen Formel II

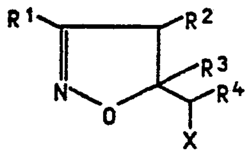

(II),

in der

$R^1$, $R^2$, $R^3$ und $R^4$ unsubstituiertes oder durch Halogen, Haloalkyl, Alkoxy, Haloalkoxy, Nitro, Cyano, Dialkylphosphonyl oder Alkoxycarbonyl substituiertes Alkyl, Aryl, Aralkyl, Alkoxycarbonyl oder Dialkoxyphosphonyl $R^2$ und $R^4$ auch Wasserstoff und $R^3$ auch Hydroxyalkyl oder durch eine abspaltbare Schutzgruppe verkapptes Hydroxyalkyl,

X Hydroxy, Halogen oder durch eine abspaltbare Schutzgruppe verkapptes Hydroxy bedeutet.

2. Verfahren zur Herstellung von Furanen der allgemeinen Formel I,

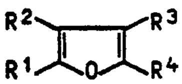

(I),

dadurch gekennzeichnet, daß man ein entsprechendes Isoxazolin der allgemeinen Formel II

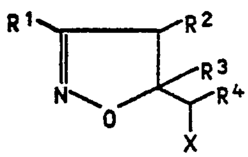

(II),

in der

$R^1$, $R^2$, $R^3$ und $R^4$ Wasserstoff oder unsubstituiertes oder durch Halogen, Haloalkyl, Alkoxy, Haloalkoxy, Nitro, Cyano, Dialkylphosphonyl oder Alkoxycarbonyl substituiertes Alkyl, Aryl, Aralkyl, Alkoxycarbonyl oder Dialkoxyphosphonyl. $R^3$ auch Hydroxyalkyl oder durch eine abspaltbare Schutzgruppe verkapptes Hydroxyalkyl und X Hydroxy, Halogen oder durch eine abspaltbare Schutzgruppe verkapptes Hydroxy bedeutet, hydriert.

3. Verfahren nach Anspruch 2, dadurch gekennzeichnet, daß man die Hydrierung unter nicht-sauren Bedingungen vornimmt, ein entsprechendes Hydroxyketon der allgemeinen Formel III

$R^1CO-CHR^2-C(OH)R^3-CH(X)R^4$ (III)

als Zwischenprodukt gewinnt und dieses durch Ansäuern in das Furan überführt.

**Claims**

1. An isoxazoline of the formula II

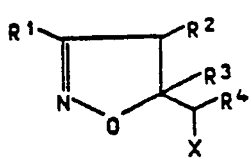

(II)

in which

$R^1$, $R^2$, $R^3$ and $R^4$ are each unsubstituted or halogen-, haloalkyl-, alkoxy-, haloalkoxy-, nitro-, cyano-, dialkylphosphonyl- or alkoxycarbonyl-substituted alkyl, aryl, aralkyl, alkoxycarbonyl or dialkoxyphosphonyl, $R^2$ and $R^4$ are each also hydrogen and $R^3$ is also hydroxyalkyl which is unblocked or blocked by a detachable protective group,

X is hydroxyl, halogen or hydroxyl blocked by a detachable protective group.

2. A process for preparing furans of the formula I

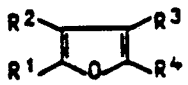

(I)

which comprises hydrogenating a corresponding isoxazoline of the formula II

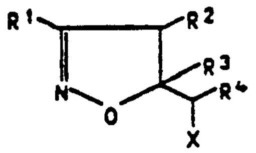

(II)

in which

$R^1$, $R^2$, $R^3$ and $R^4$ are each hydrogen or unsubstituted or halogen-, haloalkyl-, alkoxy-, haloalkoxy-, nitro-, cyano-, dialkylphosphonyl- or alkoxycarbonyl-substituted alkyl, aryl, aralkyl, alkoxycarbonyl or dialkoxyphosphonyl, $R^3$ is also hydroxyalkyl which is unblocked or blocked by a detachable protective group and

X is hydroxyl, halogen or hydroxyl blocked by a detachable protective group.

3. A process as claimed in claim 2, wherein the hydrogenation is carried out under nonacid conditions, producing a corresponding hydroxyketone of the formula III

$R^1CO\text{-}CHR^2\text{-}C(OH)R^3\text{-}CH(X)R^4$ (III)

as an intermediate which is converted by acidification into the furan.

**Revendications**

1. Isoxazoline de formule générale II

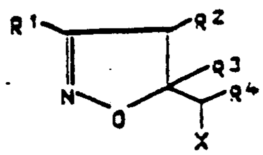

(II).

dans laquelle

$R^1$, $R^2$, $R^3$ et $R^4$ représentent chacun un groupe alkyle non substitué ou substitué par des halogènes, des groupes halogénoalkyle, alcoxy, halogénoalcoxy, nitro, cyano, dialkylphosphonyle ou alcoxycarbonyle, un groupe aryle, aralkyle, alcoxycarbonyle ou dialcoxyphosphonyle, $R^2$ et $R^4$ pouvant également représenter l'hydrogène et $R^3$ un groupe hydroxyalkyle ou un groupe hydroxyalkyle bloqué par un groupe protecteur éliminable,

X représente un groupe hydroxy, un halogène ou un groupe hydroxy bloqué par un groupe protecteur éliminable.

2. Procédé de préparation de furannes de formule générale I

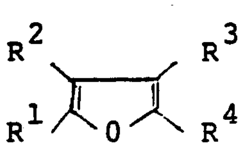

(I)

caractérisé en ce que l'on hydrogène une isoxazoline correspondante de formule générale II

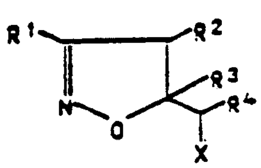

(II).

dans laquele

$R^1$, $R^2$, $R^3$ et $R^4$ représentent chacun l'hydrogène, ou un groupe alkyle non substitué ou substitué par des halogènes, des groupes halogénoalkyle, alcoxy, halogénoalcoxy, nitro, cyano, dialkylphosphonyle ou alcoxycarbonyle, un groupe aryle, aralkyle, alcoxycarbonyle ou dialcoxyphosphonyle, $R^3$ pouvant également représenter un groupe hydroxyalkyle ou un groupe hydroxyalkyle bloqué par un groupe protecteur éliminable, et

X représente un groupe hydroxy, un halogène ou un groupe hydroxy bloqué par un groupe protecteur éliminable.

3. Procédé selon la revendication 2, caractérisé en ce que l'on effectue l'hydrogénation en milieu non acide, ce qui donne en tant que produit intermédiaire une hydroxycétone correspondante de formule générale III

$$R^1CO\text{-}CHR^2\text{-}C(OH)R^3\text{-}CH(X)R^4 \quad (III)$$

qu'on convertit en le furanne par acidification.